(19) Europäisches Patentamt
European Patent Office
Office européen des brevets

(11) Veröffentlichungsnummer: **0 207 193**
**A2**

(12) # EUROPÄISCHE PATENTANMELDUNG

(21) Anmeldenummer: **85116358.4**

(22) Anmeldetag: **20.12.85**

(51) Int. Cl.4: **A61K 31/44**, //(A61K31/44,31:16)

(30) Priorität: **28.06.85 DE 3523120**

(43) Veröffentlichungstag der Anmeldung: **07.01.87 Patentblatt 87/02**

(84) Benannte Vertragsstaaten: **AT BE CH DE FR GB IT LI LU NL SE**

(71) Anmelder: **Degussa Aktiengesellschaft**
**Weissfrauenstrasse 9**
**D-6000 Frankfurt am Main 1(DE)**

(72) Erfinder: **Weischer, Carl Heinrich, Dr.**
**Schmidtbonnstrasse 8**
**D-5300 Bonn 1(DE)**
Erfinder: **Breuel, Hans-Peter, Prof.**
**Am Jungstück 30**
**D-6500 Mainz 43(DE)**
Erfinder: **Hettche, Helmut, Dr.**
**Buchrainweg 65**
**D-6050 Offenbach/Main(DE)**

(54) **Synergistische Kombination von Flupirtin und 4-Acetamido-phenol.**

(57) Arzneimittel mit synergistischer Wirkung, enthaltend eine Kombination des Analgetikums Flupirtin mit Paracetamol.

EP 0 207 193 A2

Xerox Copy Centre

## Synergistische Kombination von Flupirtin und 4-Acetamido-phenol

Flupirtin ist ein Arzneimittelwirkstoff mit analgetischen Eigenschaften. Sein chemischer Name ist 2-Amino-3-carbethoxyamino-6-(4-fluor-benzylamino)-pyridin mit der folgenden Strukturformel:

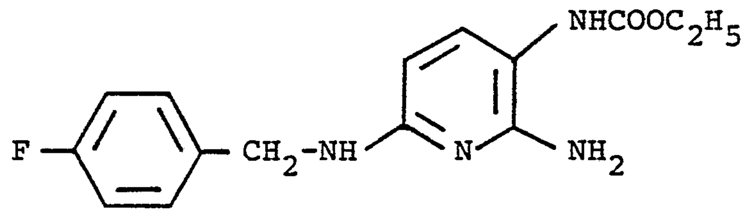

Das Flupirtin und dessen Salze mit physiologisch unbedenklichen Säuren besitzen eine ausgeprägte analgetische Hauptwirkung sowie eine geringe antiphlogistische Wirkungskomponente. Es wurde nun gefunden, daß die Wirkung des Flupirtins und seiner Salze überraschenderweise durch Kombination mit 4-Acetamido-phenol (Paracetamol) synergistisch gesteigert wird, wobei gleichzeitig die Wirkung des 4-Acetamido-phenols ebenfalls eine synergistische Steigerung erfährt. Die Wirkstoffe der erfindungsgemäßen Komtination potenzieren sich also gegenseitig in ihrer Wirkung.

Aufgabe der Erfindung ist die Bereitstellung von verbesserten Arzneimitteln mit analgetischer und anti-phlogistischer Wirkung.

Die Erfindung betrifft die durch die Patentansprüche definierten Gegenstände.

Die in den Patentansprüchen angegebenen Gewichtsmengen beziehungsweise Gewichtsteile beziehen sich jeweils auf die reinen Wirkstoffe, das heißt nicht auf Salze dieser Wirkstoffe.

Das Flupirtin wird vorzugsweise als Säureadditionssalz verwendet, wobei insbesondere die Salze mit Halogenwasserstoffsäuren (zum Beispiel das Hydrochlorid) oder organischen Säuren - (zum Beispiel das Maleat oder das Gluconat) in Frage kommen. Das Paracetamol wird im allgemeinen nicht in Form des Salzes verwendet. Falls es als Salz eingesetzt wird, handelt es sich beispielsweise um das Salz mit Alkalimetallen.

Die erfindungsgemäße Komtination zeigt beispielsweise im Randall-Selitto-Test einen Synergismus der analgetischen Wirkung, die gegenüber der Analgesie des reinen Flupirtin (Das Flupirtin wird stets als Maleat, Gluconat oder Hydrochlorid untersucht.) und dem reinen 4-Acetamido-phenol überadditiv gesteigert ist.

Es handelt sich bei der erfindungsgemäßen Kombination um einen überraschenden funktionellen Synergismus.

Die Wirkung der erfindungsgemäßen Kombination geht beispielsweise aus folgenden Versuchen hervor: Diese Untersuchungen erfolgten im Randall-Selitto-Test an der Albino-Ratte. Hierbei wird sowohl die Flupirtin-Dosis (Hydrochlorid) konstant gehalten, als auch bei konstanter Dosis des 4-Acetamido-phenols die Flupirtin-. Dosis geändert und jeweils im ersten Fall die ED50 der analgetischen Wirkung des 4-Acetamido-phenols und im zweiten Fall die analgetische Hauptwirkung des Flupirtins in der Kombination bestimmt (Methode der Bestimmung des Synergismus nach Forth, Henschler und Rummel, Lehrbuch Pharmakologie und Toxikologie, Ausgabe 1980, Seite 65; Wissenschaftsverlag, Bibliographisches Institut Mannheim, Wien und Zürich).

Die Ergebnisse zeigt die Tabelle 1:

<u>Tabelle 1:</u>

Randall-Selitto-Test nach Randall, Selitto, Arch. int. Pharmacodyn. 111, Seite 409 (1957)
Tier: Albino-Ratte (Sprague-Dawley); Applikation: per os

| Wirkstoff-Kombination | Wirkstoffdosis (mg/kg) peroral | Wirkung in % Mittel von 6 Ratten | $ED_{50}$ in mg/kg Bestimmung 30 Minuten nach Substanzgabe, Methode der linearen Regression |
|---|---|---|---|
| Flupirtin | 10,0 | -8,3 | Flupirtin |
| (Hydrochlorid) | 20,0 | 23,2 | |
| | 30,0 | 45,8 | 30,94 |
| | 40,0 | 67,5 | |
| Paracetamol | 30,0 | 38,2 | Paracetamol |
| alleine | 100,0 | 43,0 | 71,47 |
| | 300,0 | 88,1 | |
| 30,0 mg/kg | Paracetamol | | Paracetamol in der Kombination |
| Flupirtin | 0,1 | 25,5 | |
| (Hydrochlorid) | 0,3 | 44,1 | |
| + Paracetamol | 1,0 | 48,0 | 0,6 |
| | 3,0 | 65,4 | |
| | 10,0 | 99,5 | |
| 70,0 mg/kg | Flupirtin | | Flupirtin in der Kombination |
| Paracetamol | 1,0 | 41,3 | |
| + Flupirtin | 3,0 | 60,9 | |
| (Hydrochlorid) | 10,0 | 91,8 | 1,65 |
| | 30,0 | 127,3 | |

So wird beispielsweise in dem vorstehend angegebenen Test die analgetisch wirksame ED50 des Flupirtins von 30,94 mg/kg per os auf 1,65 mg/kg und die analgetisch wirksame ED50 des 4-Acetamido-phenols (Paracetamol) von 71,47 mg/kg auf 0,6 mg/kg per os gesenkt. In diesem Test erfolgt also eine Steigerung der analgetischen Wirksamkeit des Flupirtins um den Faktor 18 und hinsichtlich der analgetischen Wirkung des Paracetamols um den Faktor 119.

Die synergistische Wirkungssteigerung an der Ratte in dem oben angegebenen Test ist besonders ausgeprägt, wenn in der Kombination mindestens 30 mg Flupirtin vorliegen.

Für die erfindungsgemäßen Komtinationen kommen zum Beispiel folgende Indikationen in Betracht: Entzündliche, degenerative artikuläre und extraartikuläre rheumatische Erkrankungen, Morbus Bechterew, nichtrheumatische Entzündungs-und Schwellungszustände, nichtrheumatische Schmerzzustände, chronische Polyarthritis, Arthrosis deformans, Weichteilrheumatismus, postoperative Schmerzen, insbesondere Schmerzen nach Hals-, Nasen-und Ohren-Eingriffen, Zahnextraktionen und unfallchirurgischen Eingriffen; Schmerzen nach Traumen, insbesondere nach Frakturen, Luxationen und Distorsionen; Schmerzen bei entzündlichen Zuständen im Genitalbereich, insbesondere bei Endometritis, Adnexitis und Pelveo-peritonitis; Carzinom-Schmerzen; Dysmenorrhoe. Kontraindikationen sind beispielsweise: Gastrointestinale Beschwerden, Magen-und Darmulzera, schwer eingeschränkte Nieren-und Leberfunktion, Asthma.

Die Tagesdosen der erfindungsgemäßen Kombination bestehen zum Beispiel aus 10 bis 600 mg, vorzugsweise 50 bis 400 mg und insbesondere 100 bis 300 mg Flupirtin und etwa 4000 mg beziehungsweise 2000 oder 1000 mg, vorzugsweise 600 mg, insbesondere 200 mg Paracetamol. Bei Carcinom-Schmerzen kann die Flupirtinmenge insbesondere auch zwischen 10 bis 900 mg, vorzugsweise 50 bis 600 mg, insbesondere 100 bis 400 mg liegen. Die Tagesdosen können in Form einer einmaligen Verabreichung der gesamten Menge oder in Form von 1 bis 6, insbesondere 1 bis 4 Teildosen pro Tag eingesetzt werden. Im allgemeinen ist eine Verabreichung 1 bis 4 mal, insbesondere 1 bis 3 mal täglich bevorzugt. Beispielsweise beträgt die bevorzugte Dosis für die Kombination von Flupirtin und Paracetamol vorzugsweise 600 mg Flupirtin und etwa 4000 mg Paracetamol 1 mal täglich. Insbesondere beträgt diese Dosis etwa 300 mg Flupirtin und etwa 1500 mg Paracetamol einmal täglich.

Flupirtin und Paracetamol liegen in einer Dosierungseinheit beispielsweise in folgendem Gewichtsverhältnis vor: 1 Gewichtsteil Flupirtin wird zum Beispiel mit 1 bis 400 Gewichtsteilen Paracetamol, vorzugsweise 1 Gewichtsteil Flupirtin mit 1 bis 200 Gewichtsteilen Paracetamol, insbesondere 1 Gewichtsteil Flupirtin mit 5 bis 100 Gewichtsteilen Paracetamol kombiniert.

Beispielsweise lassen sich für die Kombination 50 bis 1000 mg Paracetamol und 5 bis 150 mg Flupirtin, vorzugsweise 100 bis 600 mg Paracetamol und 10 bis 100 mg Flupirtin, insbesondere 200 bis 400 mg Paracetamol und 20 bis 60 mg Flupirtin leicht zum Arzneimittel formulieren.

Diese zuvor angegebenen Gewichtsmengen gelten nur für homogene Mischungen von Paracetamol und Flupirtin (zum Beispiel Suppositorien oder Einschichttablette). Bei anderen Formulierungen, zum Beispiel Kapseln und Zweischichttabletten, können die Komponenten natürlich auch in anderen Gewichtsmengen zusammen kombiniert werden.

Die Dosierungseinheit der erfindungsgemäßen Kombination kann beispielsweise enthalten:

a)Bei peroralen Arzneiformen:

10 bis 300 mg Flupirtin, vorzugsweise 50 bis 250 mg, insbesondere 100 bis 200 mg Flupirtin und 1 bis 1600 mg, vorzugsweise 50 bis 1000 mg, insbesondere 100 bis 1000 mg Paracetamol.

Diese Dosen können beispielsweise 1 bis 6, vorzugsweise 1 bis 4, insbesondere 1 bis 3 mal täglich verabreicht werden.

b)Bei parenteralen Arzneiformen (zum Beispiel intravenös, instramuskulär):

10 bis 250 mg Flupirtin, vorzugsweise 20 bis 200 mg, insbesondere 50 bis 150 mg Flupirtin und 1 bis 1600 mg, vorzugsweise 50 bis 1000 mg, insbesondere 100 bis 1000 mg Paracetamol.

Diese Dosen können beispielsweise 1 bis 6, vorzugsweise 1 bis 4, insbesondere 1 bis 3 mal täglich verabreicht werden.

c)Bei Arzneiformen zur rektalen oder vaginalen Applikation:

10 bis 450 mg Flupirtin, vorzugsweise 50 bis 400 mg, insbesondere 150 bis 350 mg Flupirtin und 1 bis 1600 mg, vorzugsweise 50 bis 1000 mg, insbesondere 100 bis 1000 mg Paracetamol.

Diese Dosen können beispielsweise 1 bis 6, vorzugsweise 1 bis 4, insbesondere 1 bis 3 mal täglich verabreicht werden.

d)Bei Arzneiformen zur Applikation auf die Haut und Schleimhäute (zum Beispiel als Lösungen, Lotionen, Emulsionen, Salben, Pflaster und so weiter):

10 bis 300 mg Flupirtin, vorzugsweise 50 bis 250

mg, insbesondere 100 bis 200 mg Flupirtin und 1 bis 1600 mg, vorzugsweise 50 bis 1000 mg, insbesondere 100 bis 1000 mg Paracetamol.

Diese Dosen können beispielsweise 1 bis 6, vorzugsweise 1 bis 4, insbesondere 1 bis 3 mal täglich verabreicht werden.

Selbstverständlich können auch galenische Zubereitungen hergestellt werden, welche die oben angegebenen Dosierungseinheiten 2-bis beispielsweise 6 mal enthalten. Insbesondere enthalten Tabletten oder Kapseln der Kombination 20 bis 1800 mg, Pellets, Pulver und Granulate 20 bis 1600 mg, Suppositorien 75 bis 2600 mg an der Flupirtin-Komponente.

Die in den vorangegangenen Seiten angegebenen Dosen und Gewichtsteile, die sich auf die Anwendung am Menschen beziehen, sind jeweils bezogen auf die freien Basen, beziehungsweise freien Säuren.

Die akute Toxizität der erfindungsgemäßen Komtination an der Maus (ausgedrückt durch LD50 mg/kg; Methode: Litchfield und Wilcoxon, J. Pharmacol. Exper. Ther. 95 : 99, 1949) liegt beispielsweise für die Kombination Flupirtin (Maleat) und Paracetamol (1 : 1) bei oraler Applikation bei 734 mg/kg beziehungsweise oberhalb von 675 mg/kg Körpergewicht.

Beispiel 1

Tabletten mit 100 mg Flupirtinmaleat und 300 mg Paracetamol:

200 g Flupirtinmaleat werden mit 600 g Paracetamol, 60 g Maisstärke, 150 g Mikrokristalliner Cellulose und 60 g Modifizierter Stärke (Starch 1500/Colorcon) gemischt und die Mischung mit einer Lösung aus 22 g Gelatine in 340 g Wasser in üblicher Weise granuliert. Nach dem Trocknen wird das Granulat durch ein Sieb der Maschenweite 3,15 mm gegeben und anschließend mit 7 g Magnesiumstearat und 1 g Hochdispersem Siliciumdioxid (Aerosil 200/Degussa) gemischt. Die erhaltene Mischung wird zu Oblong-Tabletten vom Gewicht 550 mg, einer Länge von 18 mm und einer Breite von 8 mm verpreßt.

Anschließend können die Tabletten gegebenenfalls in der üblichen Weise mit einem magensaftresistenten beziehungsweise magensaftpermeablen oder -löslichen Filmüberzug versehen werden.

Eine Tablette enthält 100 mg Flupirtinmaleat sowie 300 mg Paracetamol.

Beispiel 2

Suppositorien mit 150 mg Flupirtinmaleat und 500 mg Paracetamol:

150 mg Flupirtinmaleat und 500 g Paracetamol sowie 10 mg Sojalecithin werden in 1540 g geschmolzenem Hartfett suspendiert. Nach Homogenisierung wird die Suspension in üblicher Weise in Hohlzellen von 2,3 ml ausgegossen und abgekühlt.

Ein Suppositorium vom Gewicht 2,2 g enthält 150 mg Flupirtinmaleat und 500 mg Paracetamol.

## Ansprüche

1.Arzneimittel, enthaltend als Wirkstoff Flupirtin und 4-Acetamido-phenol oder ein Salz dieser Verbindungen mit physiologisch unbedenklichen Säuren beziehungsweise physiologisch unbedenklichen Metallen.

2.Mittel nach Anspruch 1,

dadurch gekennzeichnet,

daß in der Kombination auf ein Gewichtsteil Flupirtin jeweils 1 bis 400 Gewichtsteile 4-Acetamido-phenol kommen.

3.Mittel nach einem oder mehreren der vorangegangenen Ansprüche,

dadurch gekennzeichnet,

daß in der Dosiseinheit die Kombination 10 bis 900 mg, vorzugsweise 10 bis 600 mg Flupirtin und 1 bis 1600 mg, vorzugsweise 50 bis 1000 mg 4-Acetamido-phenol enthält.

Patentansprüche für den benannten Vertragsstaat Österreich:

1.Verfahren zur Herstellung von Arzneimitteln, dadurch gekennzeichnet,

daß man als Wirkstoff Flupirtin und 4-Acetamido-phenol oder deren Salze mit physiologisch unbedenklichen Säuren beziehungsweise physiologisch unbedenklichen Metallen, gegebenenfalls mit weiteren üblichen Träger-und/oder Verdünnungsbeziehungsweise Hilfsstoffen zu pharmazeutischen Zubereitungen formuliert.

2.Verfahren nach Anspruch 1,

dadurch gekennzeichnet,

daß 1 Gewichtsteil Flupirtin mit 1 bis 400 Gewichtsteilen 4-Acetamido-phenol formuliert wird.

3.Verfahren nach einem oder mehreren der vorangegangenen Ansprüche,

dadurch gekennzeichnet,

daß 10 bis 900 mg, vorzugsweise 10 bis 600 mg Flupirtin mit 1 bis 1600 mg, vorzugsweise 50 bis 1000 mg 4-Acetamido-phenol zu einer Dosierungseinheit formuliert werden.

4.Verwendung von Flupirtin und 4-Acetamidophenol oder deren Salzen mit physiologisch unbedenklichen Säuren beziehungsweise physiologisch unbedenklichen Metallen zur Herstellung eines analgetisch wirksamen Arzneimittels.

5.Verwendung von Flupirtin und 4-Acetamidophenol oder deren Salzen mit physiologisch unbedenklichen Säuren beziehungsweise physiologisch unbedenklichen Metallen zur Herstellung eines analgetisch wirksamen Arzneimittels, in dem 1 Gewichtsteil Flupirtin mit 1 bis 400 Gewichtsteilen 4-Acetamido-phenol formuliert werden.

6.Verwendung von Flupirtin und 4-Acetamidophenol oder deren Salzen mit physiologisch unbedenklichen Saüren beziehungsweise physiologisch unbedenklichen Metallen zur Herstellung eines analgetisch wirksamen Arzneimittels, in dem 10 bis 900 mg Flupirtin mit 1 bis 1600 mg 4-Acetamidophenol zu einer Dosierungseinheit formuliert werden.